# EUROPEAN PATENT SPECIFICATION

(11) **EP 2 822 616 B1**
(45) Date of publication and mention of the grant of the patent: **20.02.2019**
(21) Application number: 13758016.3
(22) Date of filing: 06.03.2013
(51) Int. Cl.: A61M 5/158, A61L 29/14, A61M 25/00, A61M 25/06, A61L 31/08, A61L 31/10, A61L 31/14, A61L 31/16, A61M 29/00

(54) **ANTIMICROBIAL INTRODUCER AND NEEDLE**
ANTIMIKROBIELLE EINFÜHRVORRICHTUNG UND NADEL
ELÉMENT D'INTRODUCTION ET AIGUILLE ANTIMICROBIENS

(30) Priority: 06.03.2012 US 201261607512 P
(43) Date of publication of application: 14.01.2015
(73) Proprietor: PFM Medical, Inc., Carlsbad, CA 92008 (US)
(72) Inventor: KERR, Marshall, Carlsbad, CA 92008 (US)
(74) Representative: London IP Ltd
(86) International application number: PCT/US2013/029430
(87) International publication number: WO 2013/134421

(56) References cited:
- WO-A1-89/04682
- RU-C2- 2 296 587
- US-A- 5 997 815
- US-A1- 2005 004 504
- US-A1- 2008 294 111
- US-A1- 2009 287 182
- US-A1- 2009 293 882
- US-A1- 2009 293 882
- US-A1- 2009 319 035
- US-A1- 2011 009 831
- US-A1- 2011 251 559

## Description

### 1. Field of the Invention

This Application Claims Priority to U.S. Provisional Patent Application Serial Number, 61/607512 filed on March 6, 2012.

The present invention relates to an introducer and needle employed in providing intra vascular access to a patient. More specifically, the invention relates to an antimicrobial coated introducer and seldinger needle used in combination therewith, which, so coated, provides means for preventing the communication of surface area bacteria, into the insertion site.

Infection of the tissue at and adjacent to an insertion site, and/or the vascular system of a patient may easily occur when the skin of the patient is punctured during the insertion of a needle through the skin and into a vein or artery. Infectious agents such as bacteria, viruses, fungi, and other infectious agents disposed on and about the exterior surface of the skin of a patient, on conventional needles, may easily be drawn into the insertion site through a contact with the exterior surface of a needle and/or an introducer, with the skin, during the puncturing of the skin barrier. This invasion of infectious agents, can lead to inflamation and cell destruction in the tissues surrounding the puncture, or other remote infection sites should an infectious agent carried on the needle or introducer reach the patient's blood stream. The present invention provides a solution to the shortcomings of such needles and introducers through the provision of an infectious agent preventing antimicrobial coating, disposed the skin-contacting surfaces of and introducer and needle used in combination therewith, to thwart the communication of Infectious agents such as bacteria and viruses into the flex below an insertion site.

### 2. Prior Art

Document US 2009/0287181 A1 discloses an expandable transluminal sheath, for introduction into the body while in a first, small cross-sectional area configuration, and subsequent expansion of at least a part of the distal end of the sheath to a second, enlarged cross-sectional configuration. The sheath is configured for use in the vascular system and has utility in the introduction and removal of implant delivery catheters. The access route is through the femoral arteries and the iliac arteries into the aorta. The distal end of the sheath is maintained in the first, low cross-sectional configuration during advancement to the arteries into the aorta. The distal end of the sheath is subsequently expanded using a radial dilatation device, which is removed prior to the introduction of implant delivery catheters. In an exemplary application, the sheath includes a supported proximal end, a supported distal end, and a collapsible center section. Certain configurations of the sheath are capable of being inserted in a first, small cross-sectional configuration, being expanded diametrically to a second, larger cross-sectional configuration, and then being reduced to a diametrically small size for removal.

Intravascular catheters such as periphery inserted central catheters (PICC) are forms of intravenous access components which are often employed for chemotherapy regimes, antibiotic therapy, prenatal nutrition, and other treatments requiring vascular communication from outside the body of the patient. These types of catheters or conduits are often used for extended periods of time which can extend to 30 days or more. In use, the PICC, or other type catheter, must first be communicated into a vascular conduit or peripheral vein such as the cephalic vein, basilic vein, or brachial vein. Once the catheter providing the fluid conduit is inserted through the skin and into the patient's vascular system, it is advanced toward the heart to a positioning point. At this positioning point, generally, the needle tip is in a positioned communication with the cavoatrial junction.

Conventionally, insertion of the catheter through the skin and surrounding tissue of the vascular system is accomplished by a multi step process. This process is performed by a medical professional employing a plurality of medical components and instruments or tools. A commonly employed tool for piercing the skin and tissue of a patient is known as an introducer. The introducer is inserted through the skin and tissue and a distal end placed in communication with the target artery or vein of the vascular system of the patient. Once the introducer is properly engaged, it provides a conduit for the physician to easily insert the catheter axially therethrough, once the target vein has been located.

Briefly, the introducer includes a dilator including an elongated dilator body having a distal tapered region, and a tear-away sheath having an inner axial bore. The inner bore is configured to receive the dilator such that a portion of the tapered region of the dilator extends from the sheath distal end.

To properly insert and position the distal end of the catheter, for communicating and delivering the medication directly to the vascular system of the patient, first, a tourniquet or similar device is applied to the arm or leg of the patient at a position above the anticipated insertion site. This constricts blood flow thereby providing means to distend the veins so as to allow the medical professional to target a vein or artery of choice.

Subsequent to targeting the vein or artery, an introducer needle, often called a "seldinger" needle, engaged to a forward portion of a syringe, is inserted into the target vein and the tourniquet is released. The syringe is then removed and the user seals or places their thumb over the end of the needle to prevent blood loss or an intake of an air embolism.

Employing a flexible guidewire, the distal end of the guidewire is then inserted axially through the passage running through the seldinger needle until only the proximal portion of the guidewire is visible. The guidewire is advanced with a forward motion into and past the needle hub into the target vein. With the guidewire in place the needle is removed.

Next, the introducer, including sheath and dilator, is threaded over the proximal end of the guidewire and into communication with the target vein. Often a physician will nick the skin with a safety scalpel for easier advancement of the dilator. Once a proper catheter length for the determined extension into the patient has been determined, the dilator is removed from the sheath.

In a subsequent step, the distal tip of the catheter is inserted into and through the sheath until the catheter tip is correctly positioned within the target vein. The tear-away sheath is then removed by pulling it out of the vessel while simultaneously splitting the sheath. From here, adjustments can be made to the positioning of the distal end of the catheter.

As one can clearly see, there are numerous deliberate and precise actions which take place at an insertion site in such a procedure so to successfully position the catheter. Such being, namely, the insertion and removal of the needle through the protective skin layer and underlying tissue, followed by insertion and removal of the introducer through the same pathway. Although modern medicine provides means to sterilize these components and they are provided in sterile packages, there is a great chance of picking up and driving surface area pathogens and infectious agents such as bacteria and viruses, past the skin and into the flesh and vein below the insertion site. The exterior surface of the distal end of the introducer extending forward of the surrounding sheath, provides a surface carrier for such infectious agents as it passes through the skin barrier an underlying tissue and communicates through the vessel wall and into the intended blood vessel.

Such contamination can lead to the introduction of pathogens or infectious agents including one or a combination of such from a group including at least viruses, bacteria, fungi, or even parasites. This introduction transports the infectious agents past the skin barrier which had been preventing entry, and into the underlying tissue and or vein or artery of the body of the patient. For example, common bacterial skin pathogens include staphylococcus aureus and hemolytic streptococci, both of which can easily live on the skin of patients, and in the air and on various surfaces at hospitals. Herpes simplex is another common pathogen which is a viral skin disease and easily communicated into the body of the patient during such a piercing.

While attempts, modernly, have been made to increase sterilization using heat, light, and steam, and the like, there continues to be an increase in the communication of pathogens or infectious agents which thrive on the skin of patients or may be acquired on the skin of patients, into the tissues and vascular systems of patients undergoing such catheter insertion procedures.

As such, there is a continuing unmet need for a needle and introducer, configured for use in a method which provides enhanced protection against the communication of pathogens and infectious agents into the body of the patient. Such a device enabling such enhanced preventions should provide additional means beyond conventional sterilization techniques, to prevent such infectious agents from entering the underlying tissue and vascular system of a patient through an insertion site when medical devices are communicated through the skin of a patient. Such a device should provide anti pathogenic or antimicrobial surface property to exterior surfaces of the introducer and needle employed in combination therewith, to thereby extinguish or inhibit the growth of pathogens communicating the infectious agents to underlying tissue upon a contact with the respective exterior surfaces.

The forgoing examples of related art and any limitation related therewith are intended to be illustrative and not exclusive, and they do not imply any limitations on the invention described and claimed herein. Various limitations of the related art will become apparent to those skilled in the art upon a reading and understanding of the specification below and the accompanying drawings.

### SUMMARY OF THE INVENTION

The device herein disclosed and described provides a component employable in a method as a solution to the shortcomings in prior art of preventing the incursion of pathogens and infections agents through injection sites on patients. The device herein disclosed achieves the above noted goals through the provision of medical instruments employing antimicrobial surface areas having properties providing means for prevention or inhibiting the growth and/or positioning of such infectious agents on the exterior surfaces of the device.

According to the invention there is provided a device for threading a conduit into a communication with a vein or artery beneath the skin of a patient, during an insertion procedure, comprising: a hollow needle having an elongated needle body, said needle body having an exterior surface extending from a first end to a sharpened distal tip, said needle body having an interior surface defining an axial conduit communicating from said first end to said distal tip and sized for passage of a wire therethrough; a dilator having an elongated dilator body extending from a proximal end to a distal end and having an exterior surface between said proximal and distal end; said dilator body having an intermediate portion extending from said proximal end to a distal portion, said distal portion extending from said distal end toward said proximal end; said dilator body having an axial passage defined by an interior sidewall, said axial passage extending between openings at said proximal and distal end; a sheath, said sheath coaxially engageable with said dilator in an engaged position; said sheath having an elongated sheath body having and exterior surface extending from a first end to a opposite second end and having an axial passageway defined by a passage wall, said axial passageway coaxially positionable about an intermediate portion of said exterior surface of said dilator body, when in said engaged position; wherein when said dilator is in an engaged position in said sheath, the distal portion of said dilator protrudes from the distal end of said sheath, and by antimicrobial surface areas, formed upon said interior surface of said axial conduit, said exterior surface of said needle body, said passage wall of said axial passageway, said exterior surface of said sheath body, said distal portion of said dilator body, and said interior sidewall of said axial passage; and wherein said antimicrobial surface areas inhibit or prevent pathogens from positioning upon said surface areas and being communicated beneath said skin of said patient during said insertion procedure of the device.

In a first preferred mode the needle and introducer of the present invention are preferably coated from a distal end for a distance calculated to extend through the skin incision and a distance above the skin layer, with an antimicrobial area of a coating or layer on either or both the outer surface and inner surface areas. In this fashion, during any communication of the instrument through the skin, and into the patient, pathogens such as bacteria, viruses, and other infectious agents occupying the exterior and any underlying layers of skin, will come into direct contact only with the surface area bearing the antimicrobial coating.

In this mode, the device may be further sterilized using conventional heat, steam, chemical or light sterilization techniques and be subsequently sealed in sterilized packages as is commonly known in the art. For use, the devices are removed from the packages, to be provided to the user, in a conventional manner.

However, other modes are envisioned wherein an antimicrobial section or surface area of the components is provided by a means for adapting existing and stocked prepackaged and sterilized needle and introducer with the surface area of antimicrobial coating. For example, an antimicrobial coating material in the form of a liquid or vapor deposit gas may be provided which allows the user to apply the material directly to existing needle and introducer devices prior to an insertion procedure into a patient. The antimicrobial material can be provided in a spray bottle for atomizing the antimicrobial material, a salve, balm, or the like allowing a user to apply an antimicrobial coating for adapting an existing needle and introducer the advantages of such a coating. This mode of the invention provides additional utility in the art as it allows users to maintain their existing stock of sterilized needles and introducers and allows them to apply an antimicrobial coating prior to use on a patient.

In other preferred modes, the antimicrobial area of the needle and introducer is provided by the needle and introducer having surface areas which are impregnated or otherwise formed with antimicrobial materials and properties. Further, it must be noted that the antimicrobial material can be any material suitable for the intended purpose and having antimicrobial properties may be employed. Particularly favored, however, after experimentation, are one or a combination of materials in a coating or surface area extending from the distal end of the instrument which is first introduced through the skin, to a length positioning the coated surface above the skin layer once fully inserted during an insertion procedure.

Such materials can be one or a combination of antimicrobial materials, from a group of antimicrobial materials including nitrofurazone-coated silicone, silver or silver ions or silver nano-particles in a solid solution formed in combination with an adhering polymeric coating, copper or copper bearing materials in a solid solution formed in combination with an adhering polymeric coating, chlorhexidine incorporated hydroxylapatite coatings, or chlorhexidine-containing polylactide coatings. Also included in the group of antimicrobial materials can be an anodized surface having polymer and calcium phosphate coatings with chlorhexidine, viral inhibitors, fungal inhibitors, as well as known bactericides.

With respect to the above description, before explaining at least one preferred embodiment of the herein disclosed invention in detail, it is to be understood that the invention is not limited in its application to the details of construction and to the arrangement of the components in the following description or illustrated in the drawings. The invention herein described is capable of other embodiments and of being practiced and carried out in various ways which will be obvious to those skilled in the art. Also, it is to be understood that the phraseology and terminology employed herein are for the purpose of description and should not be regarded as limiting.

As such, those skilled in the art will appreciate that the conception upon which this disclosure is based may readily be utilized as a basis for designing of other structures, methods and systems for forming an instrument to communicate through the patient's skin to an interior position and project from an incision in the skin, and for carrying out the several purposes of the present disclosed device.

As used in the claims to describe the various inventive aspects and embodiments, "comprising" means including, but not limited to, whatever follows the word "comprising". Thus, use of the term "comprising" indicates that the listed elements are required or mandatory, but that other elements are optional and may or may not be present. By "consisting of" is meant including, and limited to, whatever follows the phrase "consisting of". Thus, the phrase "consisting of" indicates that the listed elements are required or mandatory, and that no other elements may be present.

It is an object of the invention to provide an instrument for communication of a pathway through the skin layer and underlying tissue of a patient, to an interior target, employing antimicrobial properties in a contact layer which has an antimicrobial coating on an exterior surface and interior surface, such as seldinger needles and introducers employed in implanting intra-vascular catheters.

It is another object of the invention to provide a means for adapting existing instruments with a user applied antimicrobial coating.

These and other objects features, and advantages of the present invention, as well as the advantages thereof over existing prior art, which will become apparent from the description to follow, are accomplished by the improvements described in this specification and hereinafter described in the following detailed description which fully discloses the invention, but should not be considered as placing limitations thereon.

### BRIEF DESCRIPTION OF DRAWING FIGURES

Figure 1 shows a view of the introducer of the present invention depicting the dilator and sheath components in an exploded view showing the intermediate portion of the introducer body which is covered by the sheath when engaged, and projecting distal portion thereof, employing a antimicrobial coating.
Figure 2 is a view of the introducer in the as used assembled mode with the dilator coaxially engaged with the sheath and the distal portion of the introducer body projecting therefrom.
Figure 3 shows a view of a seldinger needle employing a anti-microbial coating on the axial passage and exterior surface of the needle body.
Figure 4 shows a mode of the device with the introducer and needle engaged in sterile packaging.
Figure 5 shows a view of another mode of the device providing a spray bottle or other suitable means allowing the user to adapt existing instruments with an applied antimicrobial coating in an evaporative adhesive carrier.

### DETAILED DESCRIPTION OF THE PREFERRED EMBODIMENTS OF THE INVENTION

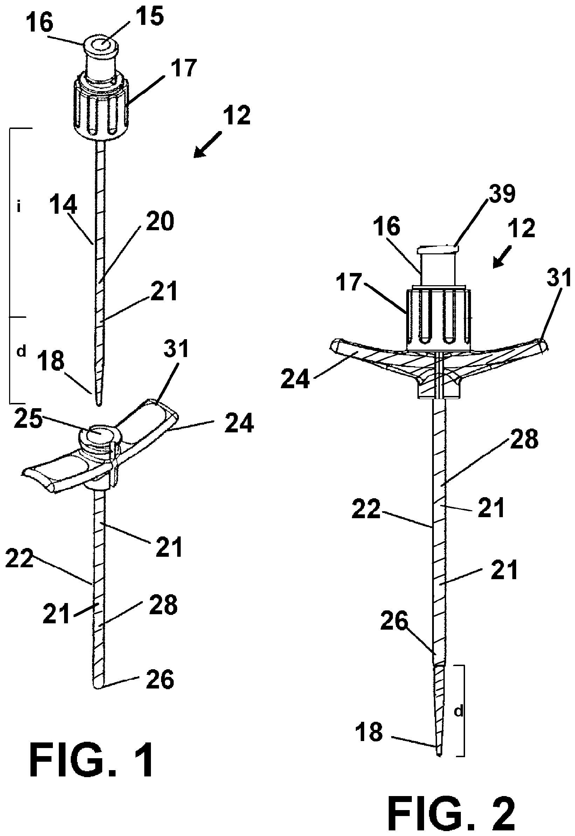

Now referring to drawings in figures 1-5, wherein similar components are identified by like reference numerals, there is seen in FIG 1 a view of an instrument for forming an initial pathway through a patient's skin layer, and underlying tissue, shown as an introducer 12, of the present invention which includes the dilator 14 and the coaxially engageable sheath 22. The various components of the device disclosed herein can be formed of metal or conventional plastic or polymeric materials adapted to the task such as a currently preferred material polytetrafluoroethylene ("PTFE"). However, it should be noted they can be formed of any material suitable for use in combination to form the disclosed surface area of anti-microbial material, for the elimination of pathogen and Infectious agents in the areas contacting or placed adjacent to flesh or skin of a patient, as noted in this disclosure.

A dilator 14 has a proximal end 16 shown with a locking nut 17 at a first end and terminating at a distal end 18 of a substantially tapered dilator body 20 component. The dilator body 20 body has an intermediate portion "I" extending from the proximal end 16 to a distal portion "d" of the dilator body 20. The dilator 14 additionally includes an interior axial passage 15 of the elongated body 20 defined by an interior sidewall surface, axially communicating from the proximal end 16, to the distal end 18. The axial passage 15 allows the introducer 12 to be translated or advanced over a pre-positioned guidewire (not shown) as noted above.

Further, as can be seen the elongated dilator body 20 of the dilator 14 features an antimicrobial surface area 21 which may be positioned on the distal portion "d" which projects from the coaxial engagement with the sheath 22 shown in figure 2. The antimicrobial surface area 21 is positioned upon all surfaces which pierce or contact the skin layer, or are positioned immediately above the skin layer of the patient and which are likely to come in contact or be placed adjacent, with that skin layer during an insertion procedure.

Again, it must be noted that the dilator body 20 can alternatively be formed itself of a solid solution of polymeric or plastic material noted which is antimicrobial impregnated with the noted antimicrobial materials herein which when cured forms a solid solution with the polymeric or plastic material forming the sheath 22 and/or deluder body 20, as a means for long term communication of antimicrobial agents thereto.

However, in other preferred modes the interior surface area of the axial passage 15, as well as a portion or all of the exterior surface of the body 20, may additionally be coated or otherwise provided a antimicrobial surface area 21 having anti-microbial properties thereon by either an impregnated coating or film, or other manner to position an antimicrobial surface area 21 on the interior surface and exterior surface. In addition, the proximal end 16 including locking nut 17 may also employ an antimicrobial surface area 21 as needed. The entire interior surface of the axial passage 15 should have the antimicrobial surface, and depending on the insertion depth of the body 20, during an insertion procedure, sufficient surface area should be covered with antimicrobial surface area 21 such that any area contacting or adjacent to skin or tissue is covered.

It is to be noted that the antimicrobial surface area 21 for all surfaces, may be applied by any conventional means known in the art such as vacuum chamber coating, plasma coating, for metal and some plastics and/or employment of an antimicrobial material or agent with a polymeric material which hardens to form a solid solution of antimicrobial impregnated polymeric material in a surface layer, or a mixture of antimicrobial material with an other adhering carrier used to form the antimicrobial surface areas 21 as would be known in the art.

Such materials include one or a combination of antimicrobial materials in a mix with the material used for forming the component itself, or, mixed in a carrier such as a polymeric material such as polyurethane or polyurethane-acrylates, which forms an adhering surface which when applied, and upon curing, is impregnated in a solid solution with and communicates antimicrobial material, to an exterior surface. Such polymeric material should have excellent adhesion and temperature resistance past 121 degrees Celsius (250 degrees Fahrenheit), when cured to form a solid solution of the polymer and antimicrobial communicating material. This allows the device to be sterilized in an autoclave or other heat type sterilization process.

For instance silver nitrate which over time produces silver ions, is combined by weight from 15 to 25% of the total mixture weight, with a polymeric material such as polyurethane or polyurethane-acrylates, and upon curing to form a solid solution coating, on any surface area to be protected, produces an excellent antimicrobial surface area 21 forming a solid solution of silver ion producing silver or silver nitrate evenly distributed withing the formed polymeric material layer.

The formed surface area 21 thus continually communicates silver ions to the areas it is placed on an instrument surface area coming in contact with skin or tissue or placed adjacent thereto. Over a long time period, the communicated silver ions provide an antimicrobial agent to eliminate bacteria, viruses, and other infectious agents in the surface area 21 of formed or coated surface which is placed anywhere the instrument contacts skin or flesh and a distance above the piercing.

Or, the formed surface area 21 may also be formed with, or include, other anti microbial materials from a group including nitrofurazone-coated silicone or copper or copper bearing materials in a curing polymer coating, and/or chlorhexidine incorporated hydroxylapatite coatings, and/or chlorhexidine-containing polylactide coatings on an anodized surface, and/or polymer and calcium phosphate coatings mixed with chlorhexidine.

The sheath 22 is shown in figure 2 in the coaxially engaged position with the dilator 14 and has a proximal end 24 shown as having the locking nut 17 and handle 31 thereon. Extending from the proximal end 24 is the elongated cylindrical sheath body 28, which communicates to an open distal end 26 from which the distal portion "d" of the dilator body 20 projects when coaxially engaged.

The sheath 22 additionally includes an axial passageway 25, defined by an interior passage wall surface of the cylindrical sheath body 28 communicating between openings at the proximal end 24 and the distal end 26. The axial passageway 25 of the sheath is intended to receive the intermediate portion "I" of the dilator body 20, therethrough in an engaged position, such that the distal portion "d" at the distal end 18 of the dilator body 20, will protrude past the distal end 26, of the sheath body 22 as shown in FIG 2.

Further, it is preferred in the present invention to provide the above noted antimicrobial surface areas 21, yielded by application of antimicrobial materials or formation of solid solutions of polymeric material and antimicrobial material forming the sheath body 28 and dilator body 20, to form an antimicrobial surface area 21, on all, or a sufficient area of the sheath body 28, and distal portion "d" which contact skin and flesh and which may extend above the skin, during an insertion procedure, in order to reduce or essentially eliminate infectious agents such as viruses or bacteria on any contact with an insertion site during operative employment of the introducer 12 in an insertion procedure.

In addition, the proximal end 24 of the sheath and the locking nut 17 and handle 31 may also be formed of plastic or polymeric infused material or have the noted anti-microbial material coating or film covering them. Further, the interior passage wall forming the passageway 25 is preferably totally additionally be coated with, film covered with, or impregnated with, or imbedded with one or a combination of the anti-microbial materials herein, to form an antimicrobial surface area 21 along the entire surface forming the axial passageway 2. As noted, the term "coated" is for convenience and is intended to include any coating, film, or surface adhered or impregnated with, the antimicrobial surface area 21, such that in all areas contacting skin or flesh or adjacent thereto, antimicrobial particles or properties are communicated thereto to prevent or minimize infectious particles and pathogens.

At a minimum, one or more surface areas from a group of surface areas should be covered with an antimicrobial surface area 21 so that areas that come into contact with the skin, or with components which contact the skin, are protected. This group of surface areas would include, the interior surface of the axial conduit 33, the exterior surface of the needle body 36, the passage wall of the axial passageway 25, the exterior surface of the sheath body 28, the distal portion "d" of the dilator body 20, and the interior sidewall of the axial passage 15.
Further included can be the intermediate portion "I" of the exterior of dilator body 20 and the handle 31, since these areas may come into contact with skin or flesh, or can come into contact with components which come into contact with skin or flesh during an insertion procedure. Of course all of the dilator body 20 surfaces, the sheath 28 surfaces, and the needle surfaces may be covered in their entirety also for maximum protection.

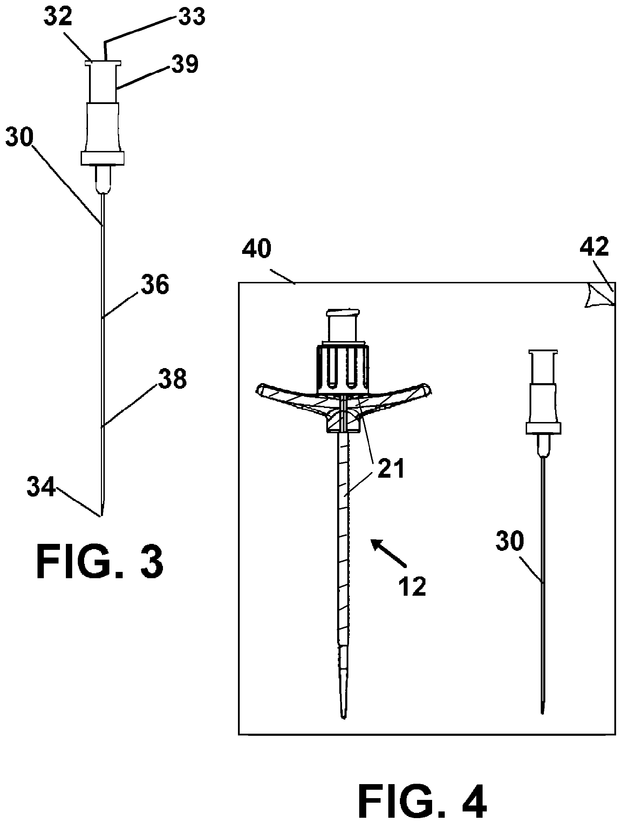

In viewing the drawings, shown in FIG 3, the seldinger needle 30 is shown as having needle body 36 with proximal end 32, with a needle hub 39 and pointed distal end 34 of a needle body 36. The needle body 36 may be formed of metal and preferably has an antimicrobial surface area 21 on all or most of the exterior surface of needle body 38 and the interior surface defining an axial conduit 33 running through the needle body 36, which as noted may be provided by the polymeric or plastic or film or vapor deposit of material having antimicrobial material therein forming an anti-microbial surface area 21 on the exterior and interior surfaces of the elongated needle body 38 as noted herein or other means providing surfaces with anti-microbial properties. It would be preferable in most cases, if cost is not an issue, to cover the entire exterior of the needle body 38 and the interior surface of the axial conduit 33 running through the needle body 38 since they are likely to always contact with, or come into contact with components which contact the patient's skin or underlying flesh.

FIG 4 shows a mode of the device 10 with the needle 30 and introducer 12 engaged within sterile packaging 40 as is conventional in the art. The packaging 40 may have a tear-away corner 42 allowing the user to open the packaging 40 and retrieve the needle 30 and introducer 12. However, it is noted that the needle 30 and introducer 12 in addition to having respective surface area may be sterilized and packaged separately.

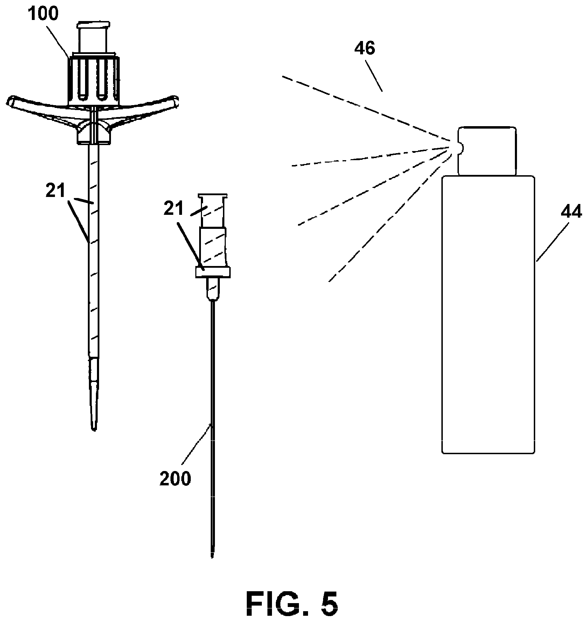

FIG 5 shows yet another mode of the disclosed device providing the user with a means for adapting existing unprotected instruments such as introducer 100 and needle 200 with an antimicrobial coating. Currently, this is provided by a spray bottle 44 or other container for discharging a mixture of a curing carrier such as polyethylene or another polymeric material which will cure when exposed to air or with addition of a curing agent. The antimicrobial material 46 mixed to dispense with the polymeric or other curing carrier, in a spray, stream, atomized spray, or the like, for the purpose of user applying an area covered with a cured solid solution of polymeric material and antimicrobial agent such as silver nitrate which will communicate silver ions to the antimicrobial surface area 21 formed by the dispensed mixture 46 on the desired unprotected surfaces of instruments 100, 200. However it is noted that other modes are envisioned wherein the antimicrobial material is provided as a liquid, gas, salve, balm, or the like which allows the user to apply a coating onto the desired instrument.

While all of the fundamental characteristics and features of the invention have been shown and described herein, with reference to particular embodiments thereof, a latitude of modification, various changes and substitutions are intended in the foregoing disclosure and it will be apparent that in some instances, some features of the invention may be employed without a corresponding use of other features without departing from the scope of the invention as set forth. It should also be understood that various substitutions, modifications, and variations may be made by those skilled in the art without departing from the scope of the invention as defined by the appended claims.

## Claims

1. A device for threading a conduit into a communication with a vein or artery beneath the skin of a patient, during an insertion procedure, comprising:
a hollow needle (30) having an elongated needle body (36), said needle body (36) having an exterior surface extending from a first end (32) to a sharpened distal tip (34), said needle body having an interior surface defining an axial conduit communicating from said first end to said distal tip and sized for passage of a wire therethrough;
a dilator (14) having an elongated dilator body (20) extending from a proximal end (16) to a distal end (18) and having an exterior surface between said proximal and distal end;
said dilator body having an intermediate portion (i) extending from said proximal end to a distal portion (d), said distal portion extending from said distal end toward said proximal end;
said dilator body having an axial passage defined by an interior sidewall, said axial passage extending between openings at said proximal and distal end;
a sheath (22), said sheath coaxially engageable with said dilator in an engaged position;
said sheath (22) having an elongated sheath body (28) having and exterior surface extending from a first end (24) to a opposite second end (26) and having an axial passageway defined by a passage wall, said axial passageway coaxially positionable about an intermediate portion of said exterior surface of said dilator body, when in said engaged position; **characterised in that** when said dilator (14) is in an engaged position in said sheath (22), the distal portion (d) of said dilator protrudes from the distal end (25) of said sheath, and by
antimicrobial surface areas, formed upon said interior surface of said axial conduit, said exterior surface of said needle body, said passage wall of said axial passageway, said exterior surface of said sheath body, said distal portion of said dilator body, and said interior sidewall of said axial passage; and
wherein said antimicrobial surface areas inhibit or prevent pathogens from positioning upon said surface areas and being communicated beneath said skin of said patient during said insertion procedure of the device.

2. The device of claim 1 additionally comprising: said antimicrobial surface area formed of one or a combination of antimicrobial materials, from a group of antimicrobial materials including, nitrofurazone-coated silicone, silver or silver ions or silver nano-particles in a solid solution formed in combination with an adhering polymeric coating, copper or copper bearing materials in a solid solution formed in combination with an adhering polymeric coating, chlorhexidine incorporated hydroxylapatite coatings, or chlorhexidine-containing polylactide coatings.

3. The device of claim 1 additionally comprising:
said antimicrobial surface area formed of silver nitrate combined by weight from 15 to 25% of a total mixture weight, with a polymeric material to form adhering polymeric coating.

4. The device of claim 3 wherein said polymeric material comprises polyurethane or polyurethane-acrylates.

5. The device of any preceding claim additionally comprising:
said sheath having a handle at said first end, at least a portion of said handle having a said antimicrobial surface area formed thereon.

6. The device of any preceding claim additionally comprising:
said antimicrobial surface having a temperature resistance to a temperature of at least
121°C (250 degrees Fahrenheit), said temperature resistance allowing said device to be heat sterilized.

## Patentansprüche

1. Eine Vorrichtung für das Einschrauben eines Rohrs in eine Verbindung mit einer Vene oder Arterie unter der Haut eines Patienten, während eines Einsetzvorgangs, umfassend:
Eine Hohlnadel (30) mit einem länglichen Nadelkörper (36), dieser Nadelkörper (36) hat eine Außenfläche, die sich von einem ersten Ende (32) zu einer angeschrägten distalen Spitze (34) erstreckt, der Nadelkörper hat eine Innenfläche, die ein axiales Rohr definiert, das von dem besagten ersten Ende zu der distalen Spitze verläuft und dimensioniert ist, um einen Draht dort hindurch zu führen;
einen Dilatator (14) mit einem länglichen Dilatatorkörper (20), der sich von einem proximalen Ende (16) zu einem distalen Ende (18) erstreckt und eine Außenfläche zwischen dem proximalen und distalen Ende hat;
dieser Dilatatorkörper hat einen Zwischenteil (i), der sich von dem proximalen Ende zu einem distalen Teil (d) erstreckt, dieser distale Teil verläuft von dem distalen Ende zu dem proximalen Ende;
der Dilatatorkörper hat einen axialen Durchgang, der durch eine innere Seitenwand definiert ist, dieser axiale Durchgang erstreckt sich zwischen den Öffnungen an dem besagten proximalen und distalen Ende;
eine Hülse (22), diese Hülse ist koaxial mit dem besagten Dilatator in einer verbundenen Position zusammenfügbar;
diese Hülse (22) hat einen länglichen Hülsenkörper (28) mit einer Außenfläche, die sich von einem ersten Ende (24) zu einem gegenüberliegenden zweiten Ende (26) erstreckt und einen axialen Durchgang, der durch eine Durchgangswand definiert ist, dieser axiale Durchgang ist koaxial positionierbar um einen Zwischenteil der besagten Außenfläche des Dilatatorkörpers, wenn er sich in der verbundenen Position befindet; **gekennzeichnet dadurch, dass**, wenn der Dilatator (14) in einer verbundenen Position in der besagten Hülse (22) ist, der distale Teil (d) des besagten Dilatators aus dem distalen Ende (25) der Hülse herausragt, und durch
antimikrobische Oberflächenbereiche, die auf der besagten Innenfläche des axialen Rohrs, der Außenfläche des Nadelkörpers, der Durchgangswand des axialen Durchgangs, der Außenfläche des Hülsenkörpers, dem distalen Teil des Dilatatorkörpers und der inneren Seitenwand des axialen Durchgangs gebildet sind; und
wobei diese antimikrobischen Oberflächenbereiche Krankheitserreger hemmen oder verhindern, dass sie sich auf den Oberflächenbereichen positionieren und während des Einsetzvorgangs der Vorrichtung unter die Haut des Patienten gelangen können.

2. Die Vorrichtung nach Anspruch 1, die zusätzlich Folgendes umfasst: den antimikrobischen Oberflächenbereich, der an einem oder einer Kombination von antimikrobischen Materialien gebildet ist, aus einer Gruppe von antimikrobischen Materialien, einschließlich Nitrofural-beschichtetem Silikon, Silber oder Silberionen oder Silber-Nanopartikeln in einer festen Lösung, gebildet in Kombination mit einer anhaftenden Polymerbeschichtung, Kupfer oder Kupfer-enthaltenden Materialien in einer festen Lösung, die in Kombination mit einer anhaftenden Polymerbeschichtung, mit Chlorhexidin verbundene Hydroxylapatitbeschichtungen oder Chlorhexidin-enthaltende Polylactidbeschichtungen gebildet sind.

3. Die Vorrichtung nach Anspruch 1, die zusätzlich Folgendes aufweist:
den antimikrobischen Oberflächenbereich, der aus Silbernitrat gebildet ist, kombiniert nach Gewicht von 15 bis 25 % eines Gesamtmischungsgewichts, mit einem Polymermaterial, um eine anhaftende Polymerbeschichtung zu bilden.

4. Die Vorrichtung nach Anspruch 3, wobei das Polymermaterial Polyurethan oder Polyurethan-Acrylate beinhaltet.

5. Die Vorrichtung nach einem der vorhergehenden Ansprüche, darüber hinaus umfassend:
Die besagte Hülse mit einem Griff an dem ersten Ende, mindestens ein Teil des Griffs hat einen der besagten antimikrobischen Oberflächenbereiche, die darauf gebildet sind.

6. Die Vorrichtung nach einem der vorhergehenden Ansprüche, darüber hinaus umfassend:
Die antimikrobische Oberfläche hat eine Temperaturbeständigkeit bis mindestens 121°C (250 Grad Fahrenheit), diese Temperaturbeständigkeit erlaubt eine Hitzesterilisation der Vorrichtung.

## Revendications

1. Dispositif destiné à enfiler un conduit en communication avec une veine ou une artère sous la peau d'un patient, pendant une procédure d'insertion, comprenant :
une aiguille creuse (30) présentant un corps d'aiguille allongé (36), ledit corps d'aiguille (36) présentant une surface extérieure s'étendant depuis une première extrémité (32) à un embout distal pointu (34), ledit corps d'aiguille présentant une surface intérieure définissant un conduit axial en communication depuis ladite première extrémité dudit embout distal et taillé pour le passage d'un fil à travers celui-ci ;
un dilatateur (14) présentant un corps de dilatateur allongé (20) s'étendant depuis une extrémité proximale (16) à une extrémité distale (18) et présentant une surface extérieure entre ladite extrémité proximale et l'extrémité distale ;
ledit corps de dilatateur présentant une partie intermédiaire (i) s'étendant depuis ladite extrémité proximale à une partie distale (d), ladite partie distale s'étendant depuis ladite extrémité distale à ladite extrémité proximale ;
ledit corps de dilatateur présentant un passage axial défini par une paroi latérale intérieure, ledit passage axial s'étendant entre les ouvertures au niveau de ladite extrémité proximale et l'extrémité distale ;
une gaine (22), ladite gaine pouvant être engagée coaxialement avec ledit dilatateur dans une position engagée ;
ladite gaine (22) présentant un corps de gaine allongé (28) présentant une surface extérieure s'étendant depuis une première extrémité (24) à une seconde extrémité opposée (26) et présentant un chemin de passage axial défini par une paroi de passage, ledit chemin de passage axial pouvant être positionné coaxialement autour d'une partie intermédiaire de ladite surface extérieure dudit corps de dilatateur, lorsqu'il est dans ladite position engagée, **caractérisé en ce que** ledit dilatateur (14) est dans une position engagée dans ladite gaine (22), la partie distale (d) dudit dilatateur dépasse depuis l'extrémité distale (25) de ladite gaine et par
des superficies antimicrobiennes, formées sur ladite surface intérieure dudit conduit axial, ladite surface extérieure dudit corps d'aiguille, ladite paroi de passage dudit chemin de passage axial, ladite surface extérieure dudit corps de gaine, ladite partie distale dudit corps de dilatateur et ladite paroi latérale intérieure dudit passage axiale ; et
dans lequel, lesdites superficies antimicrobiennes inhibent ou empêchent les agents pathogènes de se positionner sur lesdites superficies et d'être en communication sous ladite peau dudit patient pendant ladite procédure d'insertion du dispositif.

2. Dispositif selon la revendication 1, comprenant de plus : ladite superficie antimicrobienne formée d'un seul matériau ou d'une combinaison de matériaux antimicrobiens, parmi un groupe de matériaux antimicrobiens comprenant du silicone revêtu de nitrofurazone, de l'argent, des ions d'argent ou des nanoparticules d'argent dans une solution de matières solides formée en combinaison avec un revêtement polymère adhérant, du cuivre ou des matériaux porteurs de cuivre dans une solution de matières solides formée en combinaison avec un revêtement polymère adhérant, des revêtements d'hydroxylapatite incorporant de la chlorhexidine, ou des revêtements de polylactide contenant de la chlorhexidine.

3. Dispositif selon la revendication 1, comprenant de plus :
ladite superficie antimicrobienne formée de nitrate d'argent combiné en poids à partir de 15 à 25 % d'un poids de mélange total avec un matériau polymérique pour former un revêtement polymère adhérant.

4. Dispositif selon la revendication 3, dans lequel ladite matière polymère comprend du polyuréthane ou des acrylates de polyuréthane.

5. Dispositif selon une quelconque revendication précédente, comprenant de plus :
ladite gaine présentant une poignée au niveau de ladite première extrémité, au moins une partie de ladite poignée présentant une dite superficie antimicrobienne formée sur celle-ci.

6. Dispositif selon une quelconque revendication précédente, comprenant de plus :
ladite surface antimicrobienne présentant une résistance à la température à une température d'au moins 121 °C (250 degrés Fahrenheit), ladite résistance à la température permettant audit dispositif d'être stérilisé à la chaleur.
